Europäisches Patentamt

European Patent Office

Office européen des brevets

(11) Veröffentlichungsnummer: **0 366 870 B1**

(12) **EUROPÄISCHE PATENTSCHRIFT**

(45) Veröffentlichungstag der Patentschrift: **23.12.92**

(21) Anmeldenummer: **89113291.2**

(22) Anmeldetag: **20.07.89**

(51) Int. Cl.⁵: **A61M 25/01**, A61F 2/04

(54) **Ureterschiene mit Klemmverbindung zu einem Vorschubschlauch.**

(30) Priorität: **02.11.88 DE 3837196**

(43) Veröffentlichungstag der Anmeldung:
**09.05.90 Patentblatt 90/19**

(45) Bekanntmachung des Hinweises auf die
Patenterteilung:
**23.12.92 Patentblatt 92/52**

(84) Benannte Vertragsstaaten:
**AT BE CH DE ES FR GB GR IT LI NL SE**

(56) Entgegenhaltungen:
**DE-A- 2 905 703**
**DE-A- 3 446 940**
**DE-A- 3 640 745**
**DE-A- 3 736 399**
**US-A- 4 531 933**

(73) Patentinhaber: **UROMED KURT DREWS GMBH**
**Meessen 7**
**W-2000 Oststeinbek(DE)**

(72) Erfinder: **Drews, Kurt**
**Zum Osterstein 18**
**W-2000 Oststeinbek(DE)**

(74) Vertreter: **Schaefer, Konrad**
**Gehölzweg 20**
**W-2000 Hamburg 70(DE)**

Rank Xerox (UK) Business Services

# Beschreibung

Die Erfindung betrifft eine Ureterschiene der im Oberbegriff des Anspruches 1 genannten Art.

Ureterschienen dienen bei die Urindurchgängigkeit hindernden Erkrankungen des die Niere mit der Blase verbindenden Ureters zur Sicherstellung des Urinabganges aus der Niere. Sie werden mit einem durch die Harnröhre in die Blase eingeführten Endoskop von der Blase her in den Ureter bis zur Niere vorgeschoben und sind in der Regel so ausgebildet, daß sie mit freien Enden in der Niere und in der Blase liegen, die gebogen ausgeführt sind, um das Verrutschen zu verhindern. An beiden Enden sind Öffnungen vorgesehen, die den Urindurchfluß sichern. Ureterschienen sind als weiche Schläuche von wenigen Millimetern Durchmesser ausgebildet.

Zum Einführen werden diese weichen Schläuche mit einem eingelegten weniger flexiblen Mandrin (Draht bzw. Spiraldrahtschlauch oder Kombination aus beiden) so weit versteift, daß ein sicheres Vorschieben ohne Abknickgefahr auch durch Verengungen des Ureters möglich ist.

Probleme entstehen dabei dann, wenn an Engstellen des Ureters ein mehrfaches Vor- und Zurückschieben notwendig ist, um einen geeigneten Durchgang zu suchen.

Da die Schiene im wesentlichen in ihrer Länge nur von der Niere bis zur Blase reicht, also nicht durch das zum Einführen dienende Endoskop bis nach draußen, muß stets mit einem Vorschubschlauch gearbeitet werden, der ebenfalls auf dem Mandrin sitzt und das Vorschieben der Schiene ermöglicht. Die erwähnten Probleme entstehen dann, wenn außerhalb des Endoskopes zurückgezogen werden soll. Weder ein Zurückziehen am Vorschubschlauch noch ein Zurückziehen am Mandrin kann das Zurückziehen der unter Umständen fest sitzenden Schiene bewirken. Nach älterem Stand der Technik kann die Schiene also nur vorgeschoben und nicht zurückgezogen werden.

Aus dem DE-GM 86 14 013.2 ist eine Konstruktion bekannt, bei der der Vorschubschlauch mit der Ureterschiene über eine Perforation verbunden ist. Diese erlaubt beim Manipulieren während des Einführens der Ureterschiene durch Verbindung mit dem Vorschubschlauch das Vor- und Zurückziehen. Nach korrekter Verlegung der Ureterschiene muß die Perforation getrennt werden. Dazu wird am Vorschubschlauch und am Mandrin gegenläufig gezogen, wobei sich der Mandrin an der distalen Spitze der Ureterschiene, die geschlossen sein muß, abstützt. Es wird so stark gezogen, daß die Perforation zerreißt.

Aus der DE-OS 33 39 179 ist eine ähnliche Konstruktion bekannt, bei der die Ureterschiene distal auch offen sein kann. Hier wird mit dem Endoskop, also beispielsweise einer geeigneten Zange, die Ureterschiene festgehalten und dann am Vorschubschlauch bis zum Zerreißen der Perforation gezogen.

Diese Konstruktionen weisen aber eine Reihe von Nachteilen auf. So ist es schwierig, die Perforation so genau zu fertigen, daß sie beim Manipulieren hält, zum Zerreißen aber schwach genug ist. Es kommt aufgrund von Fertigungsungenauigkeiten häufig vor, daß die Perforation nicht zerrissen werden kann, ohne zu große, Verletzungsgefahren mit sich bringende Kräfte aufbringen zu müssen. Weiterhin sind am in der Blase liegenden proximalen Ende der Ureterschiene unter Umständen die Unebenheiten der Perforationsstelle störend und können beim Anliegen an der Blasenwand zu Irritationen führen. Außerdem erfordern die bekannten Vorrichtungen entweder eine Zange zum Halten der Ureterschiene oder ein geschlossenes Ende der Ureterschiene, an dem sich der Mandrin abstützt. Es ergeben sich also apparative bzw. konstruktive Einschränkungen, die unter Umständen störend sind.

Bei beiden genannten bekannten Konstruktionen besteht eine weitere apparative Beschränkung darin, daß beim Manipulieren zwar die Ureterschiene und der Vorschubschlauch verbunden sind, der Mandrin aber in seiner Position gehalten werden muß, um ein Herausrutschen zu verhindern. Zu diesem Zweck ist in der Regel am proximalen Ende des Vorschubschlauches dieser mit dem Mandrin durch eine Klemme zu verbinden. Diese erfordert zusätzliche Handhabungen beim Anlegen und Lösen.

Eine Ureterschiene der eingangs genannten Art ist in der nachveröffentlichten DE-OS 37 14 839 beschrieben. Sie löst die Probleme des erwähnten älteren Standes der Technik. Der Ansatz am Ende des Vorschubschlauches ist in das proximale Ende der Ureterschiene gesteckt und mit dem durch den Vorschubschlauch bis in die Ureterschiene vorgeschobenen Mandrin festgeklemmt. Dadurch entsteht eine für die erforderlichen Vor- und Rückziehmanipulationen ausreichende Klemmkraft, die den Vorschubschlauch an der Ureterschiene hält. Ist die Ureterschiene im Körper korrekt verlegt, so wird das außen aus dem Patienten herausragende Ende des Vorschubschlauches festgehalten und der Mandrin bis über die Klemmstelle zurückgezogen. Dadurch wird die Klemmverbindung gelöst, und der Vorschubschlauch kann herausgezogen werden.

Nachteilig bei dieser Konstruktion ist allerdings die schwierige Montagearbeit bei der Herstellung der Klemmverbindung. Diese Arbeit wird außerhalb des Patienten vorgenommen, vorteilhaft bereits in der Fabrik, so daß die Ureterschiene als über den Mandrin mit dem Vorschubschlauch verklemmte fertig handhabbare Einheit dem Arzt angeliefert wird.

Bei der Konstruktion der DE-OS 37 14 839 ist der Ansatz am Vorschubschlauch schlauchsektorartig ausgebildet und liegt in bezug auf die Schlauchachse unsymmetrisch nur zu einer Seite hin. Bei der Klemmverbindung liegt der Mandrin selbst mit einer Seite gegen die Innenseite der Ureterschiene und mit der anderen Seite gegen den Ansatz. Ist bei der Montage der Ansatz in die Ureterschiene gesteckt und wird nun der Mandrin zum Herstellen der Klemmverbindung vorgeschoben, so reibt der Mandrin einseitig gegen den Ansatz, andererseits aber gegen die Ureterschiene und versucht, bei weiterem Vorschieben in die Ureterschiene hinein durch Reibung an dieser, die Ureterschiene vorzuschieben, also vom Vorschubschlauch weg. Um dies zu verhindern, muß mit zwei Händen die Ureterschiene gegenüber dem Vorschubschlauch festgehalten werden und mit einer dritten Hand der Mandrin vorgeschoben werden. Diese Montagearbeit läßt sich also von einer Person nur schwer bewerkstelligen.

Aufgabe der vorliegenden Erfindung ist es daher, eine Ureterschiene der eingangs genannten Art zu schaffen, die unter Beibehaltung der Vorteile der Konstruktion der eingangs genannten Art bei der Manipulation im Patienten und beim Lösen der Klemmverbindung hinsichtlich der Herstellung der Klemmverbindung einfacher handhabbar ist.

Diese Aufgabe wird erfindungsgemäß mit den Merkmalen des Kennzeichnungsteiles des Anspruches 1 gelöst.

Erfindungsgemäß ist der Ansatz derart ausgebildet, daß er an der Klemmstelle den Mandrin allseitig ohne Kontakt zur Innenwand der Ureterschiene hält. Beim Vorschieben des Mandrins in die Klemmstelle hat dieser also an der Klemmstelle nur Kontakt mit dem Ansatz und drückt diesen aufweitend zur Verklemmung gegen die Ureterschiene. Der Mandrin selbst hat an dieser Stelle aber keinen Kontakt zur Ureterschiene, so daß er hier nicht unter Einwirkung der Klemmkraft stark reibend an der Ureterschiene anliegt. Beim Vorschieben des Mandrins in die Klemmstelle wird nur starke Reibkraft am Ansatz und nicht an der Ureterschiene aufgebracht. Die Ureterschiene bleibt beim Vorschieben des Mandrins auf dem Ansatz sitzen, ohne daß man sie dort festhalten muß. Es genügt also zur Montage das Arbeiten mit zwei Händen, wobei die eine Hand den Vorschubschlauch festhält und die andere Hand den Mandrin vorschiebt. Dadurch wird die Montage erheblich vereinfacht und verbilligt.

Vorteilhaft sind dabei die Merkmale des Anspruches 2 vorgesehen. Der Ansatz ist hier als gegenüber dem Rest des Vorschubschlauches reduziertes Schlauchstück ausgebildet, das den Mandrin allseitig umgibt und von diesem zur Verklemmung mit der Ureterschiene aufgeweitet wird.

Alternativ dazu ist gemäß Anspruch 3 der Ansatz vorteilhaft längsgeteilt in Form von Schlauchsektoren ausgebildet, die im wesentlichen symmetrisch zur Schlauchachse angeordnet sind, also beispielsweise zwei, drei oder vier Sektoren über den Umfang verteilt. Auch hier hat der Mandrin Kontakt nur mit den schlauchsektorförmigen Streifen des Ansatzes, ohne an der Klemmstelle unmittelbar an der Ureterschiene zu reiben.

In der Zeichnung ist die Erfindung beispielsweise und schematisch dargestellt. Es zeigen:

Fig. 1 bis 3 einen Achsschnitt durch die Verbindungsstelle mit schlauchförmigem Ansatz in unterschiedlichen Montagestellungen,

Fig. 4a bis d Schnitte nach Linie 4 - 4 in Fig. 3 mit drei Ausführungsvarianten des Ansatzes und

Fig. 5 eine Achsschnittdarstellung des Ansatzes gemäß Fig. 4d.

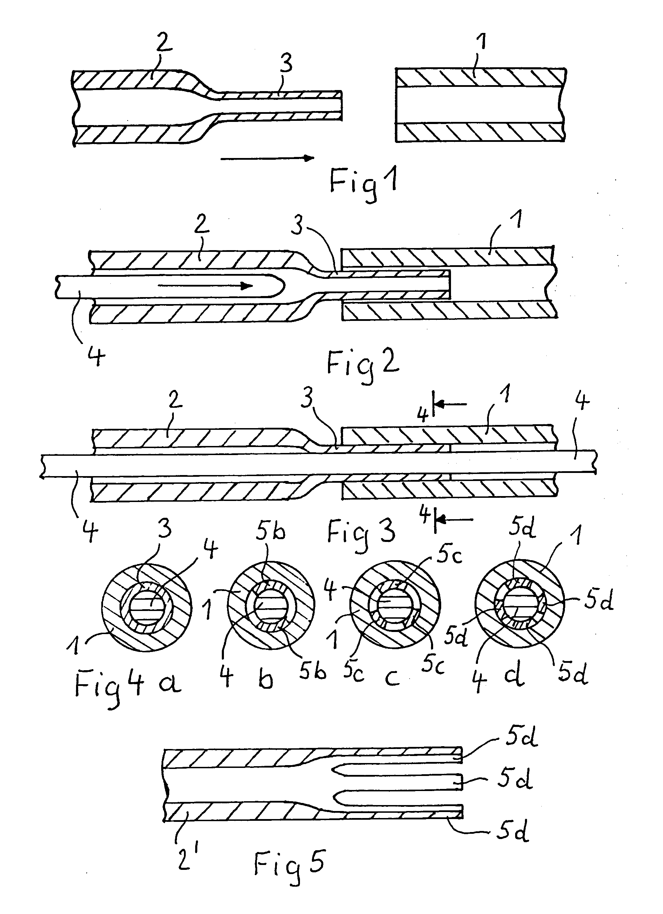

In den Fig. 1 bis 3 und 4a ist eine erste Ausführungsform der Erfindung dargestellt.

Eine Ureterschiene 1, die in den Fig. 1 bis 3 im Achsschnitt und in Fig. 4 im Querschnitt dargestellt ist, ist als weicher Kunststoffschlauch ausgebildet, der am dargestellten proximalen, also nach außen liegenden Ende offen abgeschnitten ist. Ein Vorschubschlauch 2, der in den Fig. 1 bis 3 im Achsschnitt dargestellt ist, weist an seinem dargestellten distalen Ende einen Ansatz 3 auf, der als Schlauch ausgebildet ist, welcher in seinem äußeren Durchmesser und in seinem inneren Durchmesser, wie sich aus den Fig. 1 bis 3 ergibt, gegenüber dem Vorschubschlauch 2 reduziert ist. Dabei ist der Außendurchmesser des Ansatzes 3 derart verringert, daß er mit leichtem Untermaß bzw. leicht gleitend in den Innendurchmesser der Schiene 1 paßt.

Zur Montage des Vorschubschlauches 2 an der Schiene 1 wird in Pfeilrichtung gemäß Fig. 1 der Ansatz 3 in die Schiene 1 in Axialrichtung eingesteckt, also von der noch getrennten Stellung der Fig. 1 in die eingesteckte Stellung der Fig. 2. Der Vorschubschlauch 2 mit seinem Ansatz 3 besteht ebenfalls aus flexiblem Schlauchmaterial der für diese Zwecke üblichen Art.

Ist die Einsteckstellung der Fig. 2 erreicht, so wird ein Mandrin 4 durch den Vorschubschlauch in Pfeilrichtung vorgeschoben. Der Mandrin 4 ist flexibel aus etwas steiferem Material ausgebildet, beispielsweise als Drahtwendel od. dgl.. Seine Spitze ist in geeigneter Weise verrundet, wie dargestellt.

Der Außendurchmesser des Mandrins 4 muß kleiner sein als der Innendurchmesser der Schiene 1 und größer als der Innendurchmesser des Ansatzes 3. Wird der Mandrin nun durch den Ansatz 3 bis in die Schiene 1, also in die Stellung gemäß Fig. 3 vorgeschoben, so weitet er den flexiblen Ansatz 3 derart auf, daß dieser zwischen Mandrin 4 und Schiene 1 fest eingeklemmt wird.

Wie die Fig. 4a im Querschnitt zeigt, kann der Mandrin 4 an der Klemmstelle nirgends die Schiene 1 berühren. Er steht hier nur in reibendem Kontakt mit dem Ansatz 3. Wird der Mandrin 4 weiter vorgeschoben, so ergibt sich eine Reibkraft nur gegenüber dem Ansatz 3 und somit gegenüber dem Vorschubschlauch 2. Zum Vorschieben wird das proximale Ende des Vorschubschlauches 2 mit einer Hand festgehalten und der Mandrin 4 mit der anderen Hand eingeschoben. Die Schiene 1 braucht nicht festgehalten zu werden, da beim Vorschieben der Mandrin 4 keine größere Reibkraft auf die Schiene 1 überträgt. Größere Reibkraft entsteht nur am Ansatz 3. Im weiteren Verlauf der Schiene 1 hat der Mandrin 4 ausreichendes Untermaß, um leicht und ohne Aufbringung größerer Reibkräfte gleiten zu können.

Dies ist auch von Vorteil, wenn während der Operation der Mandrin 4 gegenüber der Schiene 1 bewegt werden muß, insbesondere, was häufig vorkommt, etwas zurückgezogen werden muß. Dann braucht nur am aus dem Patienten herausragenden Ende der Vorschubschlauch 2 angefaßt und diesem gegenüber der Mandrin etwas zurückgezogen zu werden. Dies kann risikolos geschehen, ohne daß dabei die Klemmverbindung zwischen Vorschubschlauch 2 und Schiene 1 gelöst wird.

Die Anordnung gemäß Fig. 3 kann fertig montiert dem Arzt angeliefert werden. Er führt diese Anordnung in den Patienten ein, beispielsweise durch die Uretra und die Blase in einen Ureter bis zur Niere, so daß die Schiene 1 mit ihrer distalen Spitze in der Niere und mit ihrem proximalen Ende in der Blase liegt. Das proximale Ende des Vorschubschlauches 2 bleibt außerhalb des Patienten. Ist die endgültige Lage erreicht, wird der Mandrin 4 herausgezogen. Dabei löst sich die Klemmverbindung am Ansatz 3, und der Vorschubschlauch 2 kann herausgezogen werden, wobei die Schiene 1 als Harnableitverbindung zwischen Niere und Blase im Patienten liegen bleibt. Auch andere Anwendungsgebiete in der Medizin sind möglich.

In den Fig. 1 bis 4a ist der Ansatz 3 als geschlossener Schlauch ausgebildet. Es ist aber auch eine geschlitzte, in Streifen unterteilte Ausbildung des Ansatzes möglich.

Fig. 4b zeigt im Querschnitt einen Ansatz mit zwei Streifen 5b. In Fig. 4c ist eine Ausführungsform mit drei Streifen 5c dargestellt und in Fig. 4d eine Ausführung mit vier Streifen 5d.

Bei den Ausführungsformen der Fig. 4b bis 4d sind die Streifen 5b, 5c bzw. 5d jeweils im wesentlichen symmetrisch zur Schlauchachse angeordnet, um den Mandrin 4 nach allen Seiten im Abstand zur Schiene 1, also ohne Reibkontakt mit dieser zu halten.

Die geschlitzten Ausführungsformen des Ansatzes gemäß den Fig. 4b bis 4d können im Ruhezustand ähnlich wie in Fig. 1 dargestellt ausgebildet sein, also mit im Außenumfang reduziertem Durchmesser. Sie können aber auch, wie in Fig. 5 am Beispiel der Ausführungsform der Fig. 4d dargestellt, mit demselben Außendurchmesser wie der Vorschubschlauch 2'und mit größerem Innendurchmesser ausgebildet sein. Beim radialen Zusammendrücken der Streifen 5d werden diese in eine Form gebracht, die der in Fig. 1 dargestellten Form des Ansatzes 3 entspricht, so daß der aus den Streifen 5d bestehende Ansatz leicht in die Schiene 1 eingesteckt werden kann.

### Patentansprüche

1. Ureterschiene (1), die mit einem verlängernden Vorschubschlauch (2) lösbar dadurch verbunden ist, daß ein querschnittsverringerter Ansatz (3) am Ende des Vorschubschlauches in den Anfang der Ureterschiene gesteckt und mit einem durchgesteckten Mandrin (4) in der Schiene festgeklemmt ist, dadurch gekennzeichnet, daß der Ansatz (3, 5b, 5c, 5d) derart ausgebildet ist, daß an der Klemmstelle der Mandrin (4) durch den zwischenliegenden Ansatz nach allen Seiten ohne Kontakt zur Schiene (1) gehalten ist.

2. Ureterschiene nach Anspruch 1, dadurch gekennzeichnet, daß der Ansatz als gegenüber dem Vorschubschlauch (2) in Innen- und Außendurchmesser verringerter Schlauch (3) ausgebildet ist.

3. Ureterschiene nach einem der vorhergehenden Ansprüche, dadurch gekennzeichnet, daß der Ansatz längsgeteilt aus im Querschnitt schlauchsektorartig ausgebildeten Streifen (5b, 5c, 5d) besteht, die im wesentlichen symmetrisch zur Schlauchachse angeordnet sind.

### Claims

1. Ureteric catheter (1) which is releasably connected to an extending feed tube (2) by an extension (3) of reduced cross-section at the end of the feed tube being pushed into the beginning of the ureteric catheter and being clamped in the catheter with a rod (4) which is pushed through, characterised in that the extension (3, 5b, 5c, 5d) is so constructed that the rod (4) is held at the clamping point by the interposed extension on all sides without contact with the catheter (1).

2. Ureteric catheter as claimed in Claim 1, characterised in that the extension is constructed as a tube (3) of reduced internal and external diameter with respect to the feed tube (2).

3. Ureteric catheter as claimed in one of the preceding claims, characterised in that the extension comprises longitudinally separated strips (5b, 5c, 5d) of tube sector construction in cross-section, which strips are disposed substantially symmetrically to the tube axis.

**Revendications**

1. Cathéter urétéral (1) prolongé par un tuyau flexible d'avancement (2) muni d'un embout (3) de section réduite enfiché au début du cathéter auquel il est solidarisé par un mandrin (4) traversant l'embout constituant une liaison amovible, caractérisé en ce que l'embout (3, 5b, 5c, 5d) est configuré de manière à ce qu'à l'endroit du serrage, le mandrin (4) soit maintenu de tous côtés sans contact avec le cathéter (1) par l'embout intercalé.

2. Cathéter urétéral selon la revendication 1, caractérisé en ce que l'embout constitue un tuyau flexible (3) dont les diamètres extérieur et intérieur sont inférieurs à ceux du tuyau d'avancement (2).

3. Cathéter urétéral selon l'une des revendications précédentes caractérisé en ce que l'embout est subdivisé longitudinalement en bandes constituant des sections partielles de tuyau (5b, 5c, 5d) disposées de façon essentiellement symétrique par rapport à l'axe du tuyau.

Fig 1

Fig 2

Fig 3

Fig 4 a    b    c    d

Fig 5